# EUROPEAN PATENT APPLICATION

(11) **EP 4 159 106 A1**
(43) Date of publication of application: **05.04.2023**
(21) Application number: 21833569.3
(22) Date of filing: 07.06.2021
(51) Int. Cl.: A61B 1/018, A61B 1/005, A61B 1/04, A61B 1/273

(54) **ELECTRONIC ENDOSCOPE**

(30) Priority: 03.07.2020 CN 202010631476
(71) Applicant: Hangzhou Leinzett Medical Technology Co., Ltd., Zhejiang (CN)
(72) Inventor: ZHOU, Yifeng, 311115, Hangzhou, Zhejiang (CN); ZHANG, Jian, 311115, Hangzhou, Zhejiang (CN); LIU, Menghua, 311115, Hangzhou, Zhejiang (CN)
(74) Representative: FARAGO Patentanwaltsgesellschaft mbH
(86) International application number: PCT/CN2021/098683
(87) International publication number: WO 2022/001587

(57) **Abstract**

Provided is an electronic endoscope (1), comprising an insertion portion (2) and an operation portion (3) sequentially connected to each other. The insertion portion (2) comprises a front end portion (21) and a bending portion (22). The end face of a distal end of the front end portion (21) has an inclined surface (211). The inclined surface (211) crosses with the end face of the distal end of the front end portion (21) to form an edge line (216). The edge line (216) is parallel to a bending direction of the bending portion (22). The electronic endoscope (1) can achieve better delivery with low costs for use environments.

## Description

### TECHNICAL FIELD

The present invention relates to the field of medical instruments, in particular to an electronic endoscope.

### BACKGROUND

Encoscopic Retrograde Cholangio-Pancreatography (ERCP) refers to a technology for inserting a duodenoscope into a descending part of the duodenum to find the duodenal papilla, inserting an angiography catheter into an opening part of the papilla from a biopsy tube, injecting a contrast agent, and then taking an x-ray picture to show the pancreatic bile duct. On the basis of the ERCP, interventional therapies such as duodenal papillary sphincterotomy, endoscopic nasobiliary drainage, endoscopic internal biliary drainage, and endoscopic lithotomy can be carried out, which is popular among patients because of non-invasive operation and small traumas. In the above-mentioned interventional therapies, an interventional therapy instrument often needs to be threaded through an instrument channel (or referred to as a working channel, a forceps channel, etc.) of the above-mentioned duodenoscope, and enter the pancreatic bile duct through the duodenal papilla for interventional therapy. When the interventional therapy instrument is an endoscope, the above-mentioned duodenoscope can be understood as a parent endoscope.

However, endoscopes sold on the market as interventional therapy instruments are complex in structure and expensive, and can only be reused. Therefore, there is a need for an endoscope that can basically realize the above functions as an interventional therapy instrument and has low cost.

### SUMMARY

The present invention aims to solve the problem that an endoscope serving as an interventional therapy instrument has extremely high cost. The present invention provides an electronic endoscope, which can be used as an interventional therapy instrument and has lower cost.

In order to solve the above-mentioned technical problem, an embodiment of the present invention discloses an electronic endoscope, including an insertion portion and an operation portion sequentially connected to each other. The insertion portion includes a front end portion and a bending portion. The end face of a distal end of the front end portion has an inclined surface. The inclined surface crosses with the end face of the distal end of the front end portion to form an edge line. The edge line is parallel to a bending direction of the bending portion.

By the adoption of the above-mentioned technical solution, due to the arrangement of the inclined surface, the electronic endoscope provided by the present invention has good delivery in the vertical direction perpendicular to the bending direction. By means of actively controlling the bending direction, the electronic endoscope provided by the present invention has good delivery in the bending direction. Furthermore, by means of moving or rotating the operation portion, the electronic endoscope provided by the present invention can selectively use the inclined surface, or use the active control of the bending direction, or use both the inclined surface and the active control of the bending direction for cooperation according to the actual situation to achieve the delivery in different scenarios. Most importantly, the electronic endoscope in the above technical solution is simple in structure and can be manufactured at a low cost.

The above different scenarios at least include passing through a working channel (or referred to as an instrument channel or a forceps channel) of a parent endoscope or entering the duodenal papilla. That is, the electronic endoscope provided by the present invention has good delivery when passing through a parent endoscope that is implanted in a human body in advance. The electronic endoscope provided by the present invention has good delivery when entering the duodenal papilla after passing through the parent endoscope. Furthermore, by means of the cooperation with the operation portion, the advantage of the delivery is achieved by the low cost.

Optionally, the inclined surface faces a side surface of the operation portion.

Optionally, an instrument port is formed in a shell of the operation portion, and the inclined surface faces the side where the instrument port is located.

Optionally, the bending portion includes cord channels axially penetrating through the bending portion, and an intersection of an axial line of each cord channel and the end face of the distal end of the bending portion is located at an eccentric position relative to an axial line of the bending portion.

Optionally, a connecting line between the intersections of the axial lines of the cord channels and the end face of the distal end of the bending portion is parallel to the edge line.

Optionally, the front end portion includes a base; a distal end of the base is provided with a camera unit and two lighting units, and the two lighting units are respectively located on two sides of the camera unit.

Optionally, the bending portion includes an auxiliary channel, and the auxiliary channel is watertight.

Optionally, an intersection of the auxiliary channel and the end face of the distal end of the front end portion is located in an area outside the inclined surface.

Optionally, two auxiliary channels are provided; and intersections of the two auxiliary channels and the end face of the distal end of the front end portion are respectively close to the two lighting units.

Optionally, the insertion portion further includes a flexible tube portion; each of the front end portion, the bending portion and the flexible tube portion is provided with an axially run-through working channel; and the working channels of the front end portion, the bending portion and the flexible tube portion communicate with one another through pipe fittings.

### BRIEF DESCRIPTION OF THE DRAWINGS

Fig. 1 shows a front view of a structure of an electronic endoscope provided according to one embodiment of the present invention.
Fig. 2 shows a bottom view of a structure of an insertion portion provided according to one embodiment of the present invention.
Fig. 3 shows a left view of the structure of the insertion portion provided according to one embodiment of the present invention.
Fig. 4 shows a sectional view along A-A in Fig. 2.
Fig. 5 shows a bottom view of a bending state of a bending portion along a left-right direction.
Fig. 6a shows a sectional view of a junction of a front end portion and a bending portion along B-B in Fig. 3.
Fig. 6b shows a sectional view of a junction of the bending portion and a flexible tube portion along B-B in Fig. 3.
Fig. 6c shows a sectional view of a junction of the front end portion and the bending portion along C-C in Fig. 3.
Fig. 6d shows a sectional view of a junction of the bending portion and the flexible tube portion along C-C in Fig. 3.
Fig. 7a shows a three-dimensional diagram of an internal structure of an operation portion provided according to one embodiment of the present invention.
Fig. 7b shows a front view of the internal structure of the operation portion provided according to one embodiment of the present invention.
Fig. 8a shows a three-dimensional diagram of a partial internal structure of the operation portion provided according to one embodiment of the present invention.
Fig. 8b shows a front view of a partial internal structure of the operation portion provided according to one embodiment of the present invention.
Fig. 9 shows a three-dimensional diagram of a back structure of the operation portion provided according to one embodiment of the present invention.
Fig. 10 shows a three-dimensional diagram of a structure of a locking mechanism provided according to one embodiment of the present invention.
Fig. 11 shows a three-dimensional diagram of a structure of a petal-shaped block provided according to one embodiment of the present invention.
Fig. 12 shows a three-dimensional diagram of a structure of a control portion provided according to one embodiment of the present invention.
Fig. 13 shows a three-dimensional diagram of a structure of a follower portion provided according to one embodiment of the present invention.

### DETAILED DESCRIPTION

The implementation modes of the present invention are illustrated below with particular specific embodiments, and those skilled in the art can easily understand other advantages and effects of the present invention from the contents disclosed in this specification. Although the description of the present invention will be introduced in conjunction with the preferred embodiments, this does not mean that the features of the present invention are limited to this implementation mode. On the contrary, the purpose of introducing the present invention in combination with the implementation modes is to cover other options or modifications that may be extended based on the claims of the present invention. In order to provide a deep understanding of the present invention, the following description will contain many specific details. The present invention can also be implemented without using these details. In addition, in order to avoid confusing or obscuring the focus of the present invention, some specific details will be omitted in the description. It should be noted that the embodiments in the present invention and features in the embodiments may be combined with each other without conflicts.

It should be noted that in this specification, similar reference signs and letters indicate similar items in the following drawings. Therefore, once a certain item is defined in one drawing, it is unnecessary to be further defined and explained in the subsequent drawings.

In the description of this embodiment, it should be noted that orientations or positional relationships indicated by the terms "upper", "lower", "inside", "bottom" and the like are orientations or positional relationships as shown based on the drawings, or orientations or positional relationships where this invention product are usually placed, and are only for the purpose of facilitating and simplifying the description of the present invention instead of indicating or implying that devices or elements indicated must have particular orientations, and be constructed and operated in the particular orientations, so these terms cannot be construed as limiting the present invention.

In the description of this embodiment, it should be noted that the terms "proximal" and "distal" refer to relative positional relationships. When an operator operates an instrument to treat a target object, a side close to the operator along this instrument is "proximal", and a side close to the target object is "distal".

The terms "first", "second", etc. are only for the purpose of distinguishing descriptions, and may not be understood as indicating or implying the relative importance.

In the description of this embodiment, it should be also noted that unless otherwise explicitly specified and defined, the terms "arranged", "connected" and "connection" shall be understood broadly, which may be, for example, fixedly connected, or detachably connected, or integrally connected, or mechanically connected, or electrically connected, or directly connected, or indirectly connected through an intermediate medium, or internal communicating between two elements. Those of ordinary skill in the art can understand the specific meanings of the above terms in the present invention according to specific situations.

In the embodiments provided by the present invention, in order to realize some functions of an endoscope serving as an interventional therapy instrument, a bending portion and/or a flexible tube portion in an embodiment provided according to the present invention involve a plurality of channels or cavities. At this time, the description of "channel" or "cavity" is mainly relative to a bending portion and/or a flexible tube portion which are/is approximately in a solid state. If the bending portion and/or the flexible tube portion are/is not solid, for example, if they are approximately hollow, the channels or cavities involved in the bending portion and/or the flexible tube portion in this embodiment provided according to the present invention can be pipelines or paths. That is, the channels or cavities in the bending portion and/or the flexible tube portion in this embodiment provided according to the present invention can also be pipelines or paths, as long as desired paths can be provided for the corresponding components.

In order to make the objectives, the technical solutions and the advantages of the present invention clearer, detailed descriptions will be made to the implementation modes of the present invention below in combination with the accompanying drawings.

Encoscopic Retrograde Cholangio-Pancreatography (ERCP) refers to a technology for inserting a duodenoscope into a descending part of the duodenum to find the duodenal papilla, inserting an angiography catheter into an opening part of the papilla from a biopsy tube, injecting a contrast agent, and then taking an x-ray picture to show the pancreatic bile duct. On the basis of the ERCP, interventional therapies such as duodenal papillary sphincterotomy, endoscopic nasobiliary drainage, endoscopic internal biliary drainage, and endoscopic lithotomy can be carried out, which is popular among patients because of non-invasive operation and small traumas. In the above-mentioned interventional therapies, an interventional therapy instrument often needs to be threaded through an instrument channel (or referred to as a working channel, a forceps channel, etc.) of the above-mentioned duodenoscope, and enter the pancreatic bile duct through the duodenal papilla for interventional therapy. When the interventional therapy instrument is an endoscope, the above-mentioned duodenoscope can be understood as a parent endoscope.

However, endoscopes sold on the market as interventional therapy instruments are complex in structure and expensive, which brings another problem while achieving the ERCP and corresponding interventional therapies.

On August 6, 2019, the New York Times in the United States published an article entitled "Why Are These Medical Instruments So Tough to Sterilize" by Roni Caryn Rabin. The title was translated by 36 krypton (36kr. com) as "Why is it so difficult to disinfect an endoscope contacted by 500000 patients every year?". From the article: in medical institutions around the world, such a tool as a snakelike duodenoscope needs to be used to diagnose and treat diseases in the pancreatic gland and the bile duct. However, these fiber optical elements have a significant disadvantage: although they can be inserted into the upper part of the small intestine through the mouth and is reused, the fiber optical elements cannot be disinfected by conventional methods. Usually, the fiber optical elements are washed manually and then put into a dishwasher like machine for cleaning again. During the re-cleaning process, special chemicals are added to eliminate microorganisms. Even if they are cleaned strictly in accordance with the requirements, bacteria may still remain on these devices, and patients may be infected as a result. In fact, in many medical institutions in the United States and Europe, hundreds of cases in which patients have been infected by the duodenoscope have occurred. However, according to a recent test, relevant regulators seriously underestimated the risks of the infection. What is worse, these devices can even cause the spread of drug-resistant infections, and there is almost no cure plan for such infections.

Obviously, an endoscope, as an interventional therapy instrument, also has the above problems.

Therefore, there is an urgent need for an endoscope that can not only realize the above functions of diagnosing and treating diseases in the pancreatic gland and bile duct, but also avoid infections due to incomplete cleaning. In addition, it is important to hope that this endoscope is low-cost, otherwise, patients still cannot afford it. Obviously, when the cost is reduced to a certain extent, the low-cost endoscope can be used as a disposable endoscope, which will completely solve the problem of secondary infection caused by the fact that the endoscope cannot be thoroughly cleaned.

The present invention provides an electronic endoscope that can be used as an interventional therapy instrument for the above-mentioned ERCP and can achieve the above purposes, and corresponding independently manufactured and used components contained in the electronic endoscope.

In order to facilitate description of orientations, an embodiment of the present invention provides an electronic endoscope 1. A knob 3111 on an operation portion 3 is located on a front surface of the operation portion 3. An instrument port 307 is formed in the operation portion 3, and the instrument port 307 is located on a side surface of the operation portion 3. An inclined surface 211 faces the side surface of the operation portion 3. The inclined surface 211 faces one side of the instrument port 307.

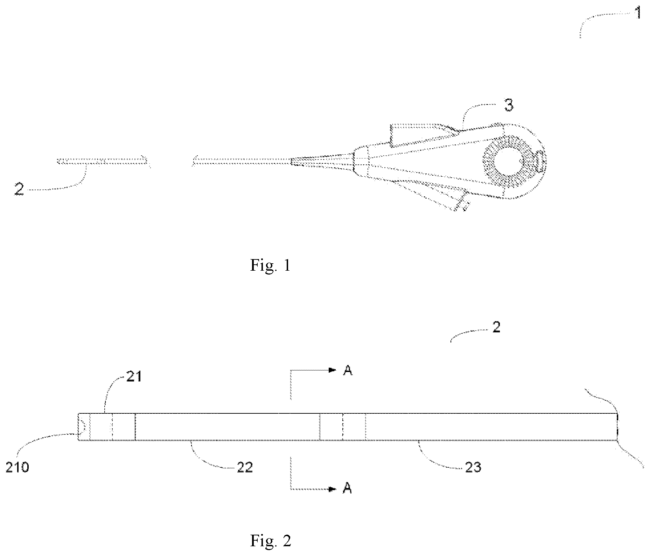

Fig. 1 shows a front view of a structure of an electronic endoscope provided according to one embodiment of the present invention. As shown in Fig. 1, the electronic endoscope 1 provided according to one embodiment of the present invention includes a slender insertion portion 2, and the operation portion 3 arranged on one side of a proximal end of the insertion portion 2. That is, the present invention provides the electronic endoscope 1, including the insertion portion 2 and the operation portion 3 which are connected to each other.

Fig. 2 shows a bottom view of a structure of an insertion portion provided according to one embodiment of the present invention. As shown in Fig. 2, the insertion portion 2 is sequentially continuously provided with a front end portion 21, a bending portion 22, and a flexible tube portion 23 from a distal end.

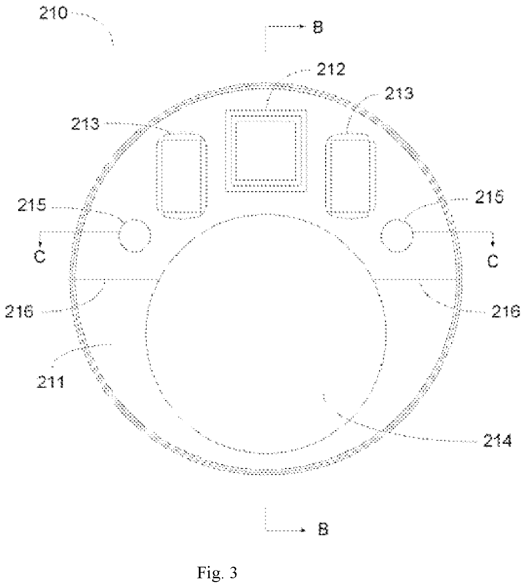

Fig. 3 shows a left view of the structure of the insertion portion provided according to one embodiment of the present invention. As shown in Fig. 3 and in combination with Fig. 1 and Fig. 2, the front end portion 21 of the insertion portion 2 is provided with a substantially cylindrical base 210. The base 210 is provided with an inclined surface 211 formed by cutting off a portion of a lower side of the end face of a distal end of the base 210. Those skilled in the art can understand that the above "cutting off" is to facilitate the visual understanding of the shape of the inclined surface 211, not to limit the formation process of the inclined surface 211.

Continuously as shown in Fig. 3, the base 210 is also provided with a plurality of cavities axially penetrating through the base 210, including a cavity for fixing a camera unit 212, a cavity for fixing lighting units 213, and a working channel 214 for allowing a working instrument to pass through. As shown in Fig. 3, when the base 210 is observed from one side of the distal end, most of the area of the working channel 214 is located in the inclined surface 211.

By the adoption of the above-mentioned technical solution, due to the arrangement of the inclined surface, the electronic endoscope provided according to the present invention has good delivery in the vertical direction perpendicular to the bending direction. By means of actively controlling the bending direction, the electronic endoscope provided according to the present invention has good delivery in the bending direction. Furthermore, by means of moving or rotating the operation portion, the electronic endoscope provided according to the present invention can selectively use the inclined surface, or use the active control of the bending direction, or use both the inclined surface and the active control of the bending direction for cooperation according to the actual situation to achieve the delivery in different scenarios. Most importantly, the electronic endoscope in the above technical solution is simple in structure and can be manufactured at a low cost. The advantage of the above low cost is embodied in various structures of the electronic endoscope provided by the present invention.

The base 210 may also include an auxiliary channel 215. The auxiliary channel 215 allows clean water to pass through. It can be understood by those skilled in the art that water is taken as an example in this embodiment, which mainly aims to intuitively explain that the auxiliary channel 215 can be provided with corresponding features according to the different objects that pass through it. That is, when the auxiliary channel 215 is used as a water channel, it should be basically sealed and able to withstand a certain pressure, which can ensure that the water is properly transported to the distal end of the base 210 and plays a normal rinsing role, instead of limiting the auxiliary channel 215 to only allow water to pass through. In this embodiment, there are two auxiliary channels 215.

In the insertion portion 2 of the electronic endoscope 1 provided by the above embodiment of the present invention, an intersection of the auxiliary channel 215 and the end face of a distal end of the front end portion 21 is located in an area outside the inclined surface 211.

The applicant finds that compared with one lighting unit, a pair of symmetrically arranged lighting units can make an image captured by the camera unit clearer and have fewer dead angles. Therefore, in this embodiment, lighting units 213 located on both sides of the camera unit 212 are still arranged on the base 210 which may be very small in size in an actual product. The applicant believes that even if the sizes of other cavities are forced to become smaller as a result, it is worth setting at least the above two lighting units 213.

In the insertion portion 2 of the electronic endoscope 1 provided by the above-mentioned embodiment of the present invention, two fixed lighting units 213 are located on both sides of the camera unit 212. Intersections of the two auxiliary channels 215 and the end face of the distal end of the front end portion 21 are respectively close to the two lighting units 213.

The base 210 should be hard. The definition of hardness here can be understood as follows: when the electronic endoscope 1 provided in this embodiment works in a human body, the base 210 should be able to withstand a corresponding pressure and overcome a corresponding resistance, so that the camera unit 212, the lighting units 213, the working channel 214 and the auxiliary channels 215 arranged on the electronic endoscope 1 can be stably maintained. The base 210 may be made of hard plastic or other materials satisfying the above conditions, such as metal, glazed ceramic and melamine resin.

In the insertion portion 2 of the electronic endoscope 1 provided by the above embodiment of the present invention, the camera unit 212 and the working channel 214 are relatively arranged at both ends of a cross section of the front end portion 21. The cross section is a section perpendicular to an axial line of the front end portion 21.

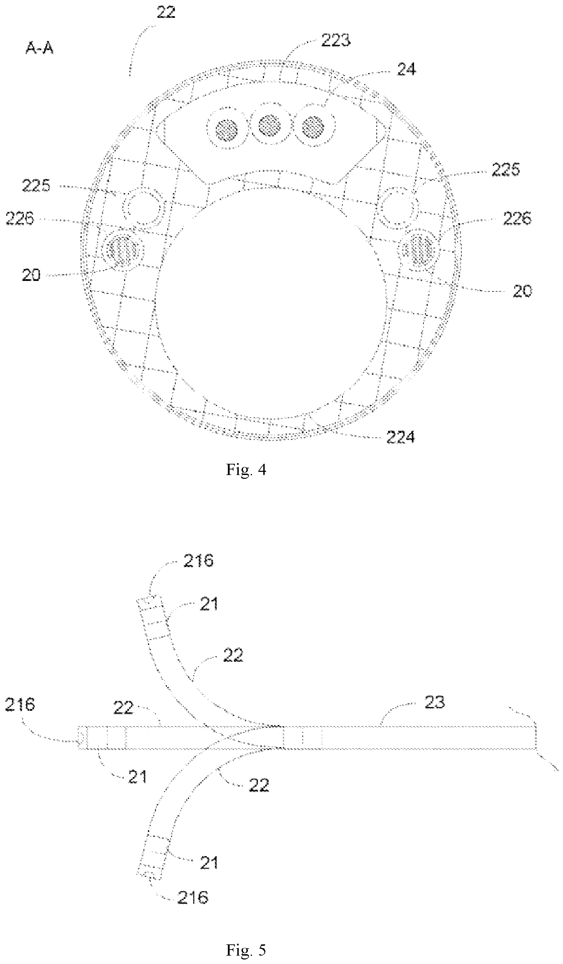

Fig. 4 shows a sectional view along A-A in Fig. 2. Fig. 4 shows a schematic sectional diagram of a bending portion 22 provided by one embodiment of the present invention. In this embodiment, the cross sections of the bending portion 22 and the flexible tube portion 23 are basically the same, and both are provided with axially run-through cavities, including a wire channel 223 and a working channel 224. According to the foregoing description, an auxiliary channel 225 may also be arranged. As previously described, the auxiliary channel 225 may be watertight.

Internal structures of the bending portion 22 and the flexible tube portion 23 correspond to an internal structure of the base 210. Specifically, wires 24 such as an electric wire and a data wire need to be provided for the camera unit 212 and the lighting units 213 arranged on the base 210 through the wire channels 223 in the bending portion 22 and the flexible tube portion 23. The wires 24 can conduct electricity in this application to supply power or transmit electronic data. For the working channel 214 formed in the base 210, the bending portion 22 and the flexible tube portion 23 also need to be provided with corresponding working channels 224, so that instruments and other objects that need to pass through the working channels 214 and 224 can pass through the flexible tube portion 23, the bending portion 22 and the base 210 from the proximal end and enter the human body or an object to be detected. When the base 210 is provided with the auxiliary channels 215, the bending portion 22 and the flexible tube portion 23 can also be provided with corresponding auxiliary channels 225 which cooperate with the auxiliary channels 215.

In the insertion portion 2 of the electronic endoscope 1 provided by the above embodiment of the present invention, the wire channel 223 and the working channel 224 are relatively arranged at both ends of a cross section of the bending portion 22. The cross section is a section perpendicular to an axial line of the front end portion 21.

Continuously as shown in Fig. 4, different from the internal structure of the base 210, the bending portion 22 and the flexible tube portion 23 are also internally provided with cord channels 226. The purpose of setting the channels is to control the bending of the bending portion 22. A cord 20 can be placed in the cord channel 226. The cord 20 passes through the operation portion 3, the flexible tube portion 23 and the bending portion 22 in sequence, starting from an operation mechanism 31 located in the operation portion 3, and is finally fixed at an eccentric position of the distal end of the bending portion 22. In this way, when an operator controls the operation mechanism 31 and tightens the cord 20, the cord 20 will act on the eccentric position of the distal end of the bending portion 22, so that the bending portion 22 bends toward the eccentric position. That is, it is an arrangement position of the cord channel 226 on the end face of the distal end of the bending portion 22 to determine a bending direction of the bending portion 22. By setting fixing points for the cord 20 at the distal end of the bending portion 22 at different eccentric positions, the bending direction of the bending portion 22 can be controlled. In order to achieve bidirectional bending of the bending portion 22, in this embodiment, the applicant sets two cords 20, and connects the proximal ends of the two cords 20 to the operation mechanism 31. The distal ends of the cords extend along the above-mentioned path and are respectively fixed at different eccentric positions of the distal end of the bending portion 22, thus achieving the bidirectional bending of the bending portion 22. In order to maximize a bending angle of the bending portion 22 in one direction, the fixing positions of the two cords 20 located at the distal end of the bending portion 22 can be opposite to each other. That is, the bending portion 22 includes two cord channels 226, and intersections of the two cord channels 226 and the end face of the distal end of the bending portion 22 are mirrored at left and right ends of the end face of the distal end of the bending portion 22 (Fig. 4). In some embodiments, the intersections can also be arranged in a center of the end face of the distal end of the bending portion 22 in an up-down direction at the same time, which is conducive to achieving a maximum bending angle of the bending portion 22. In other words, the intersections of the two cord channels 226 and the end face of the distal end of the bending portion 22 are symmetrically arranged relative to a longitudinal section that passes through the axial line of the working channel 224.

Fig. 5 shows a bottom view of a bending state of a bending portion along a left-right direction.

In the insertion portion 2 of the electronic endoscope 1 provided by the above embodiment of the present invention, the bending portion 22 includes a wire channel 223, a working channel 224 and cord channels 226 that run through the bending portion 22. An intersection of the axial line of each cord channel 226 and the end face of the distal end of the bending portion 22 is located at an eccentric position relative to the axial line of the bending portion 22. That is, the axial line of each cord channel 226 does not coincide with the axial line of the bending portion 22. When auxiliary channels 225 are set, the intersections of the two cord channels 226 and the end face of the distal end of the bending portion 22 are close to the intersections of the two auxiliary channels 225 and the end face of the distal end of the bending portion 22.

In the present invention, a connecting line between the intersections of the two cord channels 226 and the end face of the distal end of the bending portion 22 is substantially parallel to an edge line 216, which can ensure that the edge line 216 is substantially parallel to the bending direction of the bending portion 22.

When an operator controls the cord 20 to bend the bending portion 22 towards one side, the flexible tube portion 23 may also bend at a certain angle. In this embodiment, in order to make the control of the operator more accurate, a material of the flexible tube portion 23 is harder than that of the bending portion 22. In this way, when the operator controls the bending portion 22 to drive the front end portion 21 to bend through the cord 20, the flexible tube portion 23 will basically not bend, so that the position of the front end portion 21 can be easily controlled. In other embodiments, according to different purposes, the material of the flexible tube portion 23 can be the same as that of the bending portion 22, so that the flexible tube portion 23 can be controlled to follow the action of the bending portion 22 if necessary. In the above embodiment, although it is only mentioned that the bending of the bending portion 22 and the flexible tube portion 23 is controlled by means of selecting materials, those skilled in the art can understand that the bending angle of the bending portion 22 and the follower action of the flexible tube portion 23 can be controlled by controlling different specifications of the same material, or preparing different portions from different materials or the same material of different specifications. In order to make the flexible tube portion 23 harder to bend and the bending portion 22 easier to bend, on one hand, materials attached to insides and outsides of the two portions can be considered (for example, a material coats an outer side of an outer cover piece, and a material coats an inner side of an inner cover piece attached to an inner wall), and on the other hand, materials of pipelines that replace the channels or cavities can also be considered. At this time, since the pipelines are used to replace the channels or cavities, the pipelines are obviously arranged inside the bending portion 22 and/or the flexible tube portion 23. In addition, the above purpose can also be achieved by the structures of all the above components, such as a bourdon tube, in addition to the material.

In the insertion portion 2 of the electronic endoscope 1 provided by the above embodiment of the present invention, the material of the bending portion 22 or the material attached to the bending portion 22 has lower stiffness than the material of the flexible tube portion 23 or the material attached to the flexible tube portion 23, that is, the bending portion is softer and easier to bend. Or, the material or structure of a pipeline arranged in the bending portion 22 has lower stiffness than that of a pipeline arranged in the flexible tube portion 23, that is, the bending portion is softer and easier to bend. For example, a bourdon tube can be used to support the pipeline. A bourdon tube that is arranged more sparsely or has a smaller wire rod diameter is easier to bend.

When two cords 20 are provided and two cord channels 226 are arranged on the bending portion 22 and the flexible tube portion 23, one end of one entire cord 20 can be inserted into one cord channel 226 from one side of the proximal end of the flexible tube portion 23, then is sequentially threaded through the flexible tube portion 23 and the bending portion 22, and extends out of one side of the distal end of the bending portion 22 (at this time, the bending portion 22 is not connected with the front end portion 21). This end is then inserted into the other cord channel 226 from one side of the distal end of the bending portion 22 and extends out of one side of the proximal end of the flexible tube portion 23. That is, one cord 20 is threaded through the cord channels 226 of the bending portion 22 and the flexible tube portion 23 to form a U shape. An opening of the U shape is located outside the proximal end of the flexible tube portion 23, and a closed end of the U shape is located outside the distal end of the bending portion 22. The portion of the cord 20 located outside the distal end of the bending portion 22 is fixed to the end face of the distal end of the bending portion 22, for example, adhered by glue or welded, thus achieving fast mounting of the cord 20. This mounting method can significantly improve the working efficiency.

The above embodiments of present invention provide a method for mounting the cord 20 for controlling the bending of the electronic endoscope 1 on the insertion portion 2. The insertion portion 2 includes the bending portion 22 and the flexible tube portion 23. Each of the bending portion 22 and the flexible tube portion 23 is provided with two cord channels 226 that extend axially. The mounting method includes:
inserting one end of one cord 20 into one harness channel 226 from one side of the proximal end of the flexible tube portion 23;
making the above end of the cord 20 extend from the distal end of the bending portion 22;
inserting the above end of the cord 20 into the other cord channel 226 from the distal end of the bending portion 22;
making the above end of the cord 20 extend from the proximal end of the flexible tube portion 23; and
fixedly connecting a portion of the cord 20 protruding from the distal end of the bending portion 22 to the bending portion 22.

As an example in a use scenario of the embodiments provided by present invention, in the ERCP, a parent endoscope is inserted into the duodenum through an opening, and then the electronic endoscope provided by this embodiment is inserted into the duodenal papilla (a common opening of the bile duct and the main pancreatic duct in the duodenum) through an ocular endoscope for various operations. The applicant has found that the electronic endoscope provided in this embodiment needs to pass through the corresponding channels in the parent endoscope (such as a working channel, or referred to as an instrument channel, or referred to as a forceps channel) and be inserted into the duodenal papilla, which requires high delivery. Since the front end portion 21 in this embodiment is located at the frontmost end of the electronic endoscope 1 in a forwarding direction, its delivery is the most important. By practice, the applicant has found that factors affecting the delivery of the front end portion 21 mainly include the shape of the distal end of the front end portion 21 and a support of the bending portion 22 to the front end portion 21.

The shape of the distal end of the front end portion 21 is an influence factor affecting the delivery of the front end portion 21. As previously described, in this embodiment, the front end portion 21 includes a base 210. The base 210 is provided with an inclined surface 211. As shown in Fig. 3, an edge line 216 is formed at an intersection of the inclined surface 211 and the end face of the distal end of the base 210. The applicant has found that the edge line 216 can be set to be perpendicular to the axial line of the base 210 and parallel to the bending direction of the bending portion 22 on the premise of convenient operation and no increase in the cost of the electronic endoscope 1. When the electronic endoscope 1 provided in this embodiment passes through the corresponding channels of the parent endoscope, the applicant has found that the good delivery of the front end portion 21 can be achieved by selecting materials for the bending portion 22 and/or the flexible tube portion 23. At the same time, the applicant has found that in order to increase the delivery, by means of setting the structure of the operation portion 3 (see the detailed structure below), when the bending portion 22 and/or the flexible tube portion 23 adaptively bend along with the structures of the corresponding channels of the parent endoscope, they will not be improperly restrained by the cords 20. When the front end portion 21 and the bending portion 22 of the electronic endoscope 1 provided in this embodiment extend out of the parent endoscope, the bending of the bending portion 22 in a left-right direction (parallel to the edge line 216) can be achieved by controlling the aforementioned cord 20. For the bending in an up-down direction (perpendicular to the edge line 216), the bending direction of the bending portion 22 and/or the front end portion 21 around the axis can be conveniently achieved by first rotating the entire electronic endoscope 1 outside the body by a certain angle (the bending portion 22 rotates with the electronic endoscope 1) and then controlling the bending using the aforementioned cord 20. In this way, the delivery can be ensured, and the cost can be reduced.

The insertion portion 2 of the electronic endoscope 1 provided by the above embodiment of the present invention includes the front end portion 21 and the bending portion 22. The end face of the distal end of the front end portion 21 has the inclined surface 211. The inclined surface 211 crosses with the end face of the distal end of the front end portion 21 to form an edge line 216. The edge line 216 is parallel to the bending direction of the bending portion 22.

A supporting force of the bending portion 22 to the front end portion 21 is also an influence factor affecting the delivery of the front end portion 21. When the electronic endoscope 1 provided in this embodiment needs to enter a certain narrow human tissue, such as the duodenal papilla, the support to the front end portion 21 from its proximal end is important. In case of an extremely low supporting force, it is difficult for the electronic endoscope to overcome the resistance to enter the tissue successfully. The applicant has found that one of the optional solutions is to improve a connection manner between the front end portion 21 and the bending portion 22. In this embodiment, the above support is from an internal support and an external support. In fact, after a lot of experiments, the applicant has found that the purpose of the above support can be achieved by providing an internal support alone, providing an external support alone, and providing both an internal support and an eternal support at the same time.

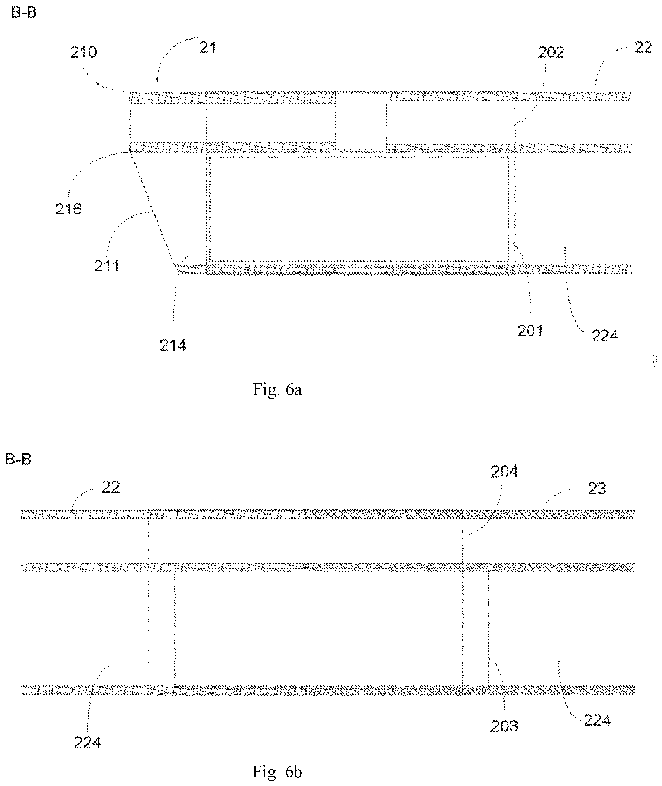

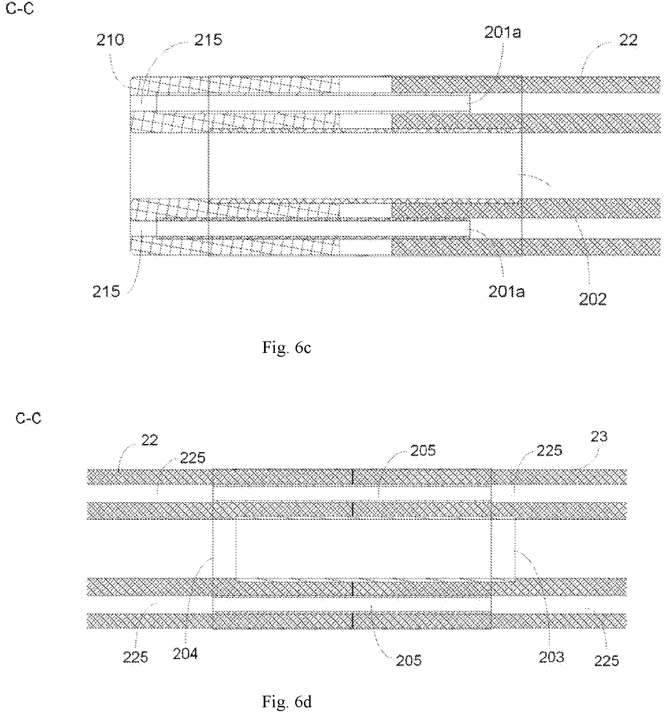

Fig. 6a shows a sectional view of a junction of a front end portion and a bending portion along B-B in Fig. 3. Fig. 6b shows a sectional view of a junction of the bending portion and a flexible tube portion along B-B in Fig. 3. Fig. 6c shows a sectional view of a junction of the front end portion and the bending portion along C-C in Fig. 3. Fig. 6d shows a sectional view of a junction of the bending portion and the flexible tube portion along C-C in Fig. 3.

As shown in Fig. 6a, the so-called internal support can be a stainless steel tube 201 with a horn mouth at its proximal end (not shown in the figure), and a distal end of the stainless steel tube is placed in the working channel 214 of the base 210. The proximal end with the horn mouth is placed in the working channel 224 of the bending portion 22, and interference fit is substantially formed, so that connection of the stainless steel tube 201 to the base 210 and the bending portion 22 is more stable. In addition, when the instrument advances from the proximal end to the distal end along the working channel, it is easier for the instrument to pass through a junction of the bending portion 22 and the base 210 due to the horn mouth. As shown in Fig. 6c, a stainless steel tube 201a with a distal end placed in the auxiliary channel 215 of the base 210 and a proximal end placed in the distal end of the bending portion 22 can also be provided, so that the role of the internal support can also be achieved on the premise of preventing water leakage between the bending portion 22 and the base 210.

The so-called external support, as shown in Fig. 6a and Fig. 6c, is a stainless steel tube 202 that is provided outside. The stainless steel tube partially surrounds the base 210 and partially surrounds the distal end of the bending portion 22. The stainless steel tube 202 hoops the base 210 and the distal end of the bending portion 22 together, which can not only prevent leakage in the junction of the base 210 and the bending portion 22, but also achieve the aforementioned purpose of supporting. In the above embodiments, during the setting of the various stainless steel tubes, it is necessary to consider the impact of the length of each stainless steel tube on the bending angle of the bending portion 22. After repeated tests, the applicant has believed that the delivery and bending control can be easily achieved simultaneously when the stainless steel tube 201 is 8 mm in length. The length of a portion that extends into the base 210 can be 4 mm or below, and can also be 2-3.5 mm. The stainless steel tube 202 may be 5-10 mm in length. When the stainless steel tube is 6 mm in length, the delivery and bending control are easily achieved simultaneously. The length of a portion that covers the base 210 and the length of a portion that covers the bending portion 22 are half of the entire length. Or, the portion that covers the base 210 may be shorter than the portion that covers the bending portion 22.

In the insertion portion 2 of the electronic endoscope 1 provided by the above embodiment of the present invention, the working channel 214 of the front end portion 21 communicates with the working channel 224 of the bending portion 22 through a pipe fitting (the stainless steel tube 201 in this embodiment). The pipe fitting (the stainless steel tube 201) that communicates the working channel 214 of the front end portion 21 with the working channel 224 of the bending portion 22 has a horn mouth which faces the bending portion 22 and is placed in the working channel 224 of the bending portion 22. The auxiliary channel 215 of the front end portion 21 communicates with the auxiliary channel 225 of the bending portion 22 through a pipe fitting (the stainless steel tube 201a in this embodiment). An outer side of the pipe fitting that communicates the auxiliary channel 215 of the front end portion 21 with the auxiliary channel 225 of the bending portion 22 is connected to the auxiliary channel 215 of the front end portion 21 and the auxiliary channel 225 of the bending portion 22 in a watertight manner. The front end portion 21 and the bending portion 22 are connected by a pipe fitting (the stainless steel tube 202 in this embodiment) that is hooped to the junction of the front end portion 21 and the bending portion 22 from the outer side.

Similarly, in order to connect the flexible tube portion 23 to the bending portion 22, or to achieve the support of the flexible tube portion 23 to the bending portion 22, the above manner of setting the internal support or the external support alone or setting both the internal support and the external support simultaneously can be adopted. Since the structures of the cross sections of the bending portion 22 and the flexible tube portion 23 are basically the same, as shown in Fig. 6b and Fig. 6d, a stainless steel tube 203 with its distal end placed in the working channel 224 at the proximal end of the bending portion 22 and its proximal end placed in the working channel 224 at the distal end of the flexible tube portion 23 can be arranged to provide the internal support. Alternatively, a stainless steel tube 205 with its distal end placed in the auxiliary channel 225 at the proximal end of the bending portion 22 and its proximal end placed in the auxiliary channel 225 at the distal end of the flexible tube portion 23 can be separately or additionally arranged. As shown in Fig. 6b and Fig. 6d, a stainless steel tube 204 that is arranged outside to provide the external support. The stainless steel tube 204 partially surrounds the proximal end of the bending portion 22 and partially surrounds the distal end of the flexible tube portion 23. The stainless steel tube 204 can be a complete steel pipe, a steel bourdon tube with an opening along an axial direction, or a steel tube or opened steel tube made of memory alloy. When the stainless steel tube 204 has an axial opening, the mounting efficiency can be improved. However, due to the existence of the opening, the dimensional accuracy of the component connected through the stainless steel tube 204 is required to be higher, otherwise the opened end portion may be warped. The human tissue or the object to be detected may be scratched after the instrument is inserted into the human body or the object to be detected, or the stability of the mounting may be affected, or an internal channel may be extruded. Therefore, the dimensional accuracy may be selected on the basis of the balance between cost and efficiency. The stainless steel tube 204 hoops the proximal end of the bending portion 22 and the distal end of the flexible tube portion 23 together. Like the foregoing internal support, the stainless steel tube 204 can not only prevent the leakage in the junction, but also achieve the purpose of supporting. Similarly, the lengths of the stainless steel tubes 203, 204 and 205 need to be considered to avoid the impact on the bending angles of the bending portion 22 and the flexible tube portion 23. After repeated tests, the applicant has believed that the stainless steel tube 203 may be 5-10 mm in length, which easily achieves the delivery and bending control simultaneously, and about half of the stainless steel tube 203 extends into the bending portion 22. The stainless steel tube 204 may be 5-12 mm in length, which easily achieves the delivery and bending control simultaneously, and about half of the stainless steel tube 204 covers the bending portion 22. The stainless steel tube 205 may be 5-10 mm in length, which easily achieves the delivery and bending control simultaneously, and about half of the stainless steel tube 205 extends into the bending portion 22.

In addition, in this embodiment, the flexible tube portion 23 and the bending portion 22 are also internally provided with cord channels 226, so a stainless steel tube 206 that is partially located in the cord channel 226 at the proximal end of the bending portion 22 and partially located in the cord channel 226 at the distal end of the flexible tube portion 23 can be arranged, which can not only ensure that it is smooth enough to control the cord 20 to pass through the junction, but also achieve the aforementioned effect of supporting. The size of the stainless steel tube 206 may be the same as that of the stainless steel tube providing the internal support.

In the insertion portion 2 of the electronic endoscope 1 provided by the above embodiment of the present invention, the working channel 224 of the bending portion 22 communicates with the working channel 224 of the flexible tube portion 23 through a pipe fitting (the stainless steel tube 203 in this embodiment). The cord channel 226 of the bending portion 22 communicates with the cord channel 226 of the flexible tube portion 23 through a pipe fitting (the stainless steel tube 206 in this embodiment). The auxiliary channel 225 of the bending portion 22 communicates with the auxiliary channel 225 of the flexible tube portion 23 through a pipe fitting (the stainless steel tube 205 in this embodiment). The outer side of the pipe fitting (the stainless steel tube 205 in this embodiment) communicating the auxiliary channel 225 of the bending portion 22 with the auxiliary channel 225 of the flexible tube portion 23 is connected to the auxiliary channel 225 of the bending portion 22 and the auxiliary channel 225 of the flexible tube portion 23 in a watertight manner. The bending portion 22 and the flexible tube portion 23 are connected by a pipe fitting (the stainless steel tube 204 in this embodiment) that is hooped to the junction of the bending portion 22 and the flexible tube portion 23 from the outer side.

Both the bending portion 22 and the flexible tube portion 23 are made of flexible materials. The flexible materials may be of a same kind, or the material of the bending portion 22 is softer than the material of the flexible tube portion 23. In this embodiment, they are manufactured by an extrusion process.

In the embodiments provided by the present invention, the above flexible materials may be Pebax^{®} elastomer (a polyether amide block copolymer composed of a rigid polyamide block and a flexible polyether block). All the above stainless steel tubes can be fixedly connected with the flexible materials by bonding. The stainless steel tube arranged outside may also be fixedly connected to the portion that is hooped inside by using a press grip process alone (with a press grip machine) or by combining a press grip process with a bonding process. In addition, the mutual connection between the flexible materials may also be achieved by hot melting. When the flexible materials are connected with each other by hot melting, the flexible materials which are subjected to hot melting and then cooling can provide sufficient supporting themselves under an appropriate process. At this time, no stainless steel tubes that provide the internal support or the external support can be arranged between the front end portion 21 and the bending portion 22, and between the bending portion 22 and the flexible tube portion 23. That is, the fixed connections between the front end portion 21 and the bending portion 22, as well as between the bending portion 22 and the flexible tube portion 23, can be achieved through the hot melting process, the internal and external supports required above can also be provided. Of course, after the hot melting, a rough surface will be formed on an outer surface of the flexible material sometimes. After the instrument enters the human body, this rough surface may cause various problems. Sometimes the flexible materials connected together by the hot melting cannot withstand repeated bending during operation and break at the junction. Therefore, those skilled in the art can understand that, in summary, a hot melting process with lower cost can be used to replace the stainless steel tubes and the bonding process or press grip process applicable to the stainless steel tubes and flexible materials. However, under the comprehensive consideration of the support, the rough surface and the connection strength, the above various processes and structures can be applied or combined in different parts as required.

It is worth noting that in this embodiment, the internal and external supports between the front end portion 21, the bending portion 22 and the flexible tube portion 23 are all the stainless steel tubes. Those skilled in the art can understand that the stainless steel tubes are only an optional way. Supporting members made of other materials, such as polylactic acid, can also be used, as long as the supporting members can satisfy the strength required by the above internal and external supports and meet the requirements for the rustproofness and surfaces required by the instrument entering the human body or the object to be detected.

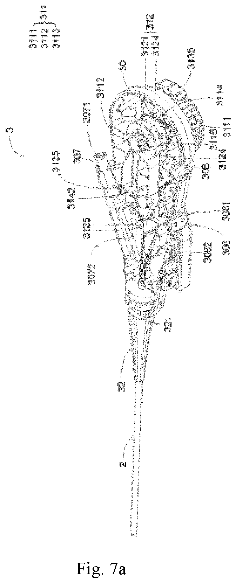

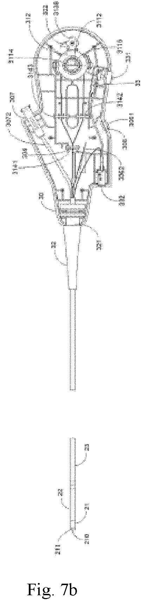

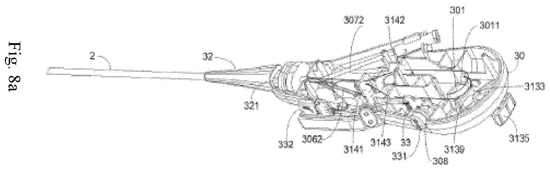

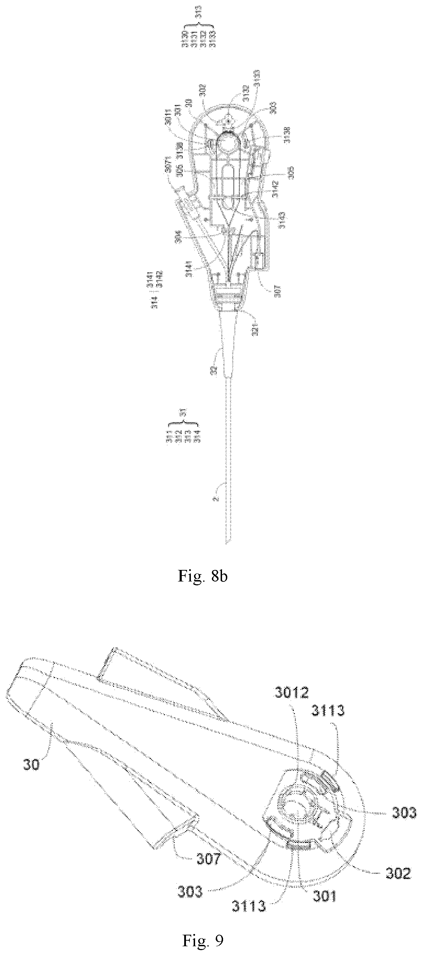

Fig. 7a shows a three-dimensional diagram of an internal structure of an operation portion provided according to one embodiment of the present invention. Fig. 7b shows a front view of the internal structure of the operation portion provided according to one embodiment of the present invention. Fig. 8a shows a three-dimensional diagram of a partial internal structure of the operation portion provided according to one embodiment of the present invention. Fig. 8b shows a front view of a partial internal structure of the operation portion provided according to one embodiment of the present invention.

In order to make the illustration clearer, there is no control portion 311 and no follower portion 312 in Fig. 8a and Fig. 8b relative to Fig. 7a and Fig. 7b.

As shown in Fig. 7a and Fig. 7b, the insertion portion 2 and the operation portion 3 are connected through a sheath 32. The sheath 32 is roughly conical, and a narrow portion 321 is arranged in the middle. The insertion portion 2 is allowed to pass through an inner cavity of the sheath 32, that is, the proximal end of the insertion portion 2 extends into the operation portion 3 and protrudes from the end face of the proximal end of the sheath 32.

As shown in Fig. 7b, a bottom end of the sheath 32 is H-shaped, and the narrow portion of the H-shaped portion is clamped to the corresponding protruding portion on a shell to achieve assembling. The narrow portion 321 in the middle of the sheath 32 may also be clamped to the corresponding protruding portion of a shell 30 to achieve assembling.

As shown in Fig. 7a, Fig. 7b, Fig. 8a and Fig. 8b, the operation portion 3 may be composed of the shell 30 with an internal cavity. In this embodiment, the shell 30 is provided with an auxiliary port 306, an instrument port 307, a button port 308 and a knob port 301. When the operator holds the operation portion 3 with a left hand and finally controls the bending portion 22 with a right hand by means of controlling the corresponding component on the operation portion 3, an opening of the sheath 32 is generally downward relative to the operator, and the instrument port 307 is arranged on a front side of the operation portion 3, with an opening facing diagonally upward. The knob port 301 is located on a right side. The auxiliary port 306 is arranged at a rear side of the operation portion 3, with an opening facing diagonally upward. The knob port 301 is circular, and is provided with a circular ring. An axial line of the circular ring coincides with that of the knob port 301, and the knob port 301 extends towards the inner and outer sides of the shell 30 of the operation portion 3 respectively to form flanges. The flange extending inwards is defined as an inner flange 3011, and the flange extending outwards is defined as an outer flange 3012. The operation portion 3 may be used for being held when the operator operates the electronic endoscope provided by the present invention.

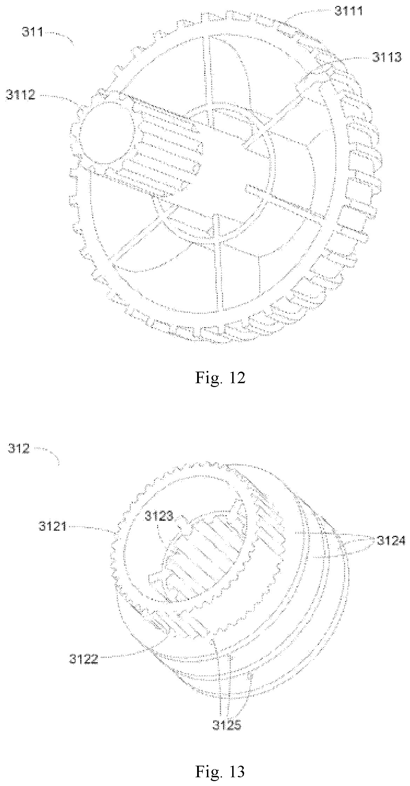

The operation portion 3 includes an operation mechanism 31. The operation mechanism 31 further includes a control portion 311, a follower portion 312, a locking component 313, and a cord restraining component 314. With reference to Fig. 12, in this embodiment, the control portion 311 includes a knob 3111 and a rotating shaft 3112. The knob 3111 is arranged outside the shell 30, and a limit block 3113 is arranged on the end face of an inward side of the knob 3111. An axial line of the rotating shaft 3112 coincides with that of the knob 3111. One end of the rotating shaft 3112 is fixed to the knob 3111, and the other end is peripherally provided with teeth and is defined as a toothed end. As shown in Fig. 9, two other limit blocks 3113 are arranged on the outer side of the shell 30 and correspond to the limit block 3113 arranged on the end face of the inner side of the knob 3111.

With reference to Fig. 13, the follower portion 312 includes a follower ring 3121. The follower ring 3121 is approximately a hollow cylinder and is arranged in the shell 30. An end, close to the knob port 301, of the follower ring 3121 is peripherally provided with external teeth and is defined as an outer toothed end 3122. An inner circumference of one end away from the knob port 301 is provided with internal teeth and is defined as an inner toothed end 3123. An inner diameter of the outer toothed end 3122 of the follower ring 3121 is larger than that of the inner flange 3011 formed by the inward extension of the knob port 301, so that the outer toothed end 3122 of the follower ring 3121 may sleeve the inner flange 3011 and be rotatable. The follower ring 3121 is provided with three annular sheets 3124 that protrude radially from the circumference, and each annular sheet 3124 is provided with a positioning hole 3125. In this embodiment, the positioning holes 3125 formed in different annular sheets 3124 overlap each other in a circumferential position, that is, the axes of the positioning holes 3125 formed in different annular sheets 3124 coincide.

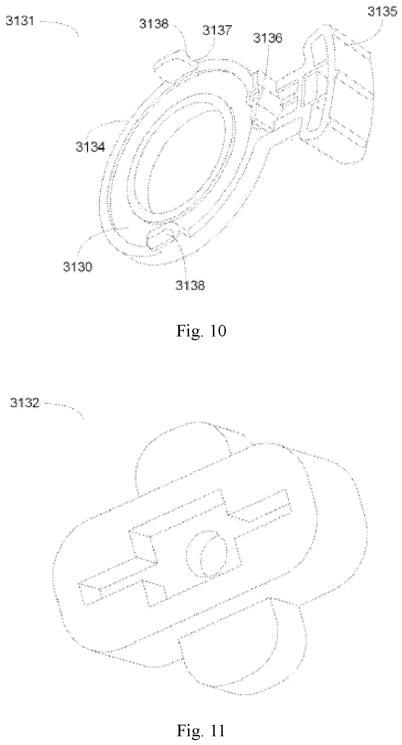

The locking component 313 includes a locking mechanism 3131, a petal-shaped block 3132, and a locking block 3133. With reference to Fig. 10, the locking mechanism 3131 further includes a circular ring 3134, a handle 3135 and a cross block 3136.

In this embodiment, the circular ring 3134, the handle 3135 and the cross block 3136 are fixedly connected with each other. As shown in the figure, the handle 3135 is arranged on one side of the circular ring 3134, and is roughly placed on the same plane as the circular ring 3134, and the cross block 3136 is arranged on the handle 3135 and extends toward the shell 30. With reference to Fig. 9, the shell 30 is provided with a cross slot 302 corresponding to the cross block 3136. The cross block 3136 extends into the shell 30 through the cross slot 302 and is fixedly connected with the petal-shaped block 3132 located in the shell 30. In this embodiment, fast and stable mounting between the cross block 3136 and the petal-shaped block 3132 is achieved by setting a slot that can be clamped to the cross block 3136 in an outward end of the petal-shaped block 3132. An edge of the circular ring 3134 is provided with a positioning block 3137. The positioning block 3137 extends from an outer edge of the end face of the inner side (the side facing the shell 30) of the circular ring 3134 to the inner side (the shell 30), and a step 3138 projecting outward relative to an axial line of the circular ring 3134 is formed at an extending tail end.

In the operation portion 3 of the electronic endoscope 1 provided by the above embodiments of the present invention, the cross block 3136 and the positioning block 3137 of the locking mechanism 3131 are located on the same side of the circular ring 3134.

The locking block 3133 is arranged on an inner wall of the shell 30 and extends into the shell 30. A side, facing the knob port 301, of the locking block 3133 is provided with a sharp bulge 3139.

In addition, with reference to Fig. 9, the shell 30 is also provided with a slot 303. The positioning block 3137 arranged at the edge of the circular ring 3134 can extend into the above slot 303 respectively, and the circular ring 3134 may be assembled on the shell 30 by clamping the step 3138 protruding from the tail end of the positioning block 3137 to the side of the slot 303. In this way, according to the restriction of the slot 303 on the positioning block 3137 and the restriction of the cross slot 302 on the cross block 3136, the locking mechanism 3131 may be assembled on the shell 30 and may rotate a certain angle with respect to the shell 30. In order to make the rotation of the circular ring 3134 smoother, in this embodiment, an inner ring of the circular ring 3134 sleeves the outer flange 3012 of the knob port 301. Otherwise, the rotation of the circular ring 3134 may only depend on the cooperation between the positioning block 3137 and the slot 303, which may cause undesirable deflection to affect the use.

The mutual positional relationship between the above components is as follows: the toothed end of the rotating shaft 3112 passes through the outer flange 3012 of the knob port 301, the inner flange 3011 of the knob port 301, the outer toothed end 3122 of the follower ring 3121, and the inner toothed end 3123 of the follower ring 3121 in sequence, is meshed with the inner toothed end 3123 of the follower ring 3121, and protrudes from the end face of the inner toothed end 3123 of the follower ring 3121 along the axial direction. At this time, due to the arrangement of the outer flange 3012, the limit block 3113 on the end face of the inner side of the knob 3111 may rotate with the knob 3111 without touching the shell 30. When the knob 3111 rotates, the limit block 3113 on the shell 30 controls the maximum rotating angle of the knob 3111 by limiting a moving range of the limit block 3113 on the end face of the inner side of the knob 3111.

Therefore, those skilled in the art can understand that in a non-locked state, the operator turns the knob 3111, and the knob 3111 drives the rotating shaft 3112 fixedly connected with the knob 3111 to rotate. The rotating shaft 3112 drives the follower ring 3121 to rotate around the inner flange 3011.

When the operator intends to achieve locking, the operator moves the handle 3135. The handle 3135 drives the circular ring 3134 to rotate. The circular ring 3134, the positioning block 3137, and the cross block 3136 respectively rotate a certain angle along the restrictions of the outer flange 3012, the slot 303, and the cross slot 302. At this time, as shown in Fig. 8b, the cross block 3136 drives the petal-shaped block 3132 in the shell 30 to move along the cross slot 302, and the protruding portion of the petal-shaped block 3132 extrudes the locking block 3133, so that the locking block 3133 elastically deforms, and the sharp bulge 3139 is clamped to or engaged with the outer toothed end 3122 of the follower ring 3121, making the follower ring 3121 unable to rotate, thereby achieving the locking.

In other embodiments, the toothed end of the rotating shaft 3112 is provided with a pin hole 3114 that penetrates radially through the rotating shaft 3112, and a pin 3115 that passes through the pin hole 3114 is arranged, which can prevent the follower ring 3121 from being axially separated from the rotating shaft 3112.

In other embodiments, the locking component 313 further includes an annular groove 3130 for placing an elastic washer. In a state that rotatable fit is completed, the elastic washer is placed in the annular groove 3130 and located between the circular ring 3134 and the shell 30. The elastic washer 3130 can reduce noise made when the circular ring 3134 rotates relative to the shell 30. In a locked state, the contact between the circular ring 3134 and the shell 30 can be damped by increasing a friction force, so that the connection is more stable.

Those skilled in the art can understand that in the present invention, for the components named as the cross block and the petal-shaped block, the descriptions of cross and petal are for the convenience of understanding their functions, rather than defining their specific shapes. They all belong to the protection scope of this application as long as they can achieve a transmission effect that is desired to be achieved in this application. Continuously as shown in Fig. 7a, Fig. 7b, Fig. 8a and Fig. 8b, as previously described, in this embodiment, the bending of the bending portion 22 is controlled by tightening and loosening the two cords 20 at the proximal end. That is, the distal end of the cord 20 is fixed at the distal end of the bending portion 22, and the proximal end of the cord 20 passes through the bending portion 22, the flexible tube portion 23, and the sheath 32 in sequence to enter the shell 30 of the operation portion 3, passes through the cord restraining component 314 in sequence in the shell 30 of the operation portion 3, passes through at least two of the positioning holes 3125 formed in the annular sheets 3124 of the follower ring 3121, and is fixed to the positioning hole 3125 which the proximal end finally passes through by welding or bonding. The applicant has found that the proximal end of the cord 20 can be positioned in a way of first passing through the positioning hole 3125 in one annular sheet 3124 and finally passing through and being fixed to the positioning hole 3125 in another annular sheet 3124, which can not only achieve the effect of fast mounting, but also achieve stable positioning of the proximal end of the cord 20, and will not affect the action of the operation mechanism 31. In particular, after the cord 20 passes through at least two different positioning holes 3125, a thin rod can be threaded through all the positioning holes. For further fastening, the positioning holes 3125 can be glued, which can not only achieve fast mounting, but also achieve stable positioning of the proximal end of the cord 20, and will not affect the action of the operation mechanism 31.

In the operation portion 3 of the electronic endoscope 1 provided by the above embodiments of the present invention, the proximal end of the cord 20 passes through the positioning hole 3125 of the middle annular sheet 3124 of the follower ring 3121.

In the operation portion 3 of the electronic endoscope 1 provided by the above embodiments of the present invention, a thin rod is arranged on the follower ring and is used for being inserted into the positioning hole 3125 through which the cord 20 passes and fixing the proximal end of the cord 20 to the positioning hole 3125.

In this embodiment, the cord restraining component 314 includes a main wire passing plate 3141 and two branch wire passing plates 3142. The main wire passing plate 3141 is arranged on distal sides of the branch wire passing plates 3142, and the branch wire passing plates 3142 are arranged on a proximal side of the main wire passing plate 3141. Two positioning holes 3125 are formed in the middle of the main wire passing plate 3141, and are respectively used for allowing two cords 20 to pass through. An edge portion of the main wire passing plate 3141 is provided with an insertion groove with a downward opening, which can be inserted to an insertion plate 304 fixed on the inner wall of the shell 30 to achieve fast fixation of the position of the main wire passing plate 3141 with respect to the shell 30. The two branch wire passing plates 3142 are respectively arranged in two cord passages 305. Each branch wire passing plate 3142 is provided with one positioning hole 3125. An insertion groove 3143 is formed in an inner wall of each cord passage 305. The branch wire passing plates 3142 are fixed on the shell 30 through the insertion grooves 3143 inserted to the inner walls of the cord passages 305. Those skilled in the art can understand that higher mounting efficiency can be achieved by first threading the cords 20 through the positioning holes 3125 and then inserting and fixing the main wire passing plate 3141 and the branch wire passing plates 3142.

In the operation portion 3 of the electronic endoscope 1 provided by the above embodiments of the present invention, the shell 30 is provided with the cord passage 305. The cord passage 305 is provided with the main wire passing plate 3141 and/or the branch wire passing plates 3142. The main wire passing plate 3141 and/or the branch wire passing plates 3142 are provided with the positioning holes 3125. The main wire passing plate 3141 is provided with an upper positioning hole 3125 and a lower positioning hole 3125.

In addition, as previously described, in order to make the insertion portion 2 easier to pass through the corresponding channels of the parent endoscope, that is, to improve the delivery of the insertion portion 2 relative to the corresponding channels of the parent endoscope serving as a use environment, the structure of the operation portion 3 can also be configured so that when the bending portion 22 and/or the flexible tube portion 23 bend adaptively with the structures of the corresponding channels of the parent endoscope, they will not be restrained improperly by the cords 20. The applicant has found that in order to prevent the cords 20 from being too tight, which would hinder the above delivery, it is necessary to keep the cords 20 loose to a certain extent. At this time, due to the structural arrangement that the cords 20 pass through the positioning holes 3125 in the main wire passing plate 3141 and the branch wire passing plates 3142, or the structural arrangement that the cords 20 pass through the positioning holes 3125 in the annular sheets 3124, when the cords 20 are loose, there will be no interference or confusion between the cords 20 and between the cords 20 and the surrounding components. In this way, during the operation, the cords 20 can quickly enter a working state and function normally only if the knob 3111 is turned.

In the operation portion 3 of the electronic endoscope 1 provided by the embodiments of the present invention, the length of the cord 20 arranged in the shell 30 is greater than a path of the cord 20 in the shell 30.

As previously described, the shell 30 of the operation portion 3 is also provided with an auxiliary port 306 and an instrument port 307. As previously described, the insertion portion 2 extends to the proximal end to enter the shell 30 of the operation portion 3 and protrudes from the end face of the proximal end of the sheath 32. As previously described, it is the flexible tube portion 23 of the insertion portion 2 that protrudes from the end face of the proximal end of the sheath 32 into the shell 30, and is internally provided with a working channel 224, a wire channel 223, and a cord channel 226. In other embodiments, an auxiliary channel 225 can also be provided.

The instrument port 307 is provided with a Luer taper 3071, which communicates with the working channel 224 of the flexible tube portion 23 through a stainless steel tube 3072. The auxiliary port 306 is provided with a sheath 3061, which communicates with the auxiliary channel 225 of the flexible tube portion 23 through a stainless steel tube 3062 (the communicating state between the auxiliary port 306 and the stainless steel tube 3062 is not shown in the figure).

In other embodiments, the shell 30 of the operation portion 3 is also internally provided with an electronic control unit 33. A button 331 of the electronic control unit 33 protrudes from the button port 308 on the shell 30 out of the shell 30 for use by the operator. The shell 30 of the operation portion 3 may also be provided with an electronic output port 332. The electronic control unit 33, the camera unit 212, the lighting units 213, and the electronic output port 332 achieve required power supplying and data transmission through the wires 24.

Although the present invention has been illustrated and described by referring to some preferred implementation modes of the present invention, those of ordinary skill in the art should understand that the above content is a further detailed description of the present invention in conjunction with specific implementation modes and cannot be considered that the specific implementations of the present invention are only limited to these descriptions. Those skilled in the art can make various changes in forms and details, including several simple deductions or substitutions, without departing from the spirit and scope of the present invention.

## Claims

1. An electronic endoscope, comprising an insertion portion and an operation portion sequentially connected to each other, wherein the insertion portion comprises a front end portion and a bending portion; the end face of a distal end of the front end portion has an inclined surface; the inclined surface crosses with the end face of the distal end of the front end portion to form an edge line; and the edge line is parallel to a bending direction of the bending portion.

2. The electronic endoscope according to claim 1, wherein the inclined surface faces a side surface of the operation portion.

3. The electronic endoscope according to claim 1, wherein an instrument port is formed in a shell of the operation portion, and the inclined surface faces the side where the instrument port is located.

4. The electronic endoscope according to claim 1, wherein the bending portion comprises cord channels axially penetrating through the bending portion, and an intersection of an axial line of each cord channel and the end face of the distal end of the bending portion is located at an eccentric position relative to an axial line of the bending portion.

5. The electronic endoscope according to claim 4, wherein a connecting line between the intersections of the axial lines of the cord channels and the end face of the distal end of the bending portion is parallel to the edge line.

6. The electronic endoscope according to claim 1, wherein the front end portion comprises a base; a distal end of the base is provided with a camera unit and two lighting units, and the two lighting units are respectively located on two sides of the camera unit.

7. The electronic endoscope according to claim 1, wherein the bending portion comprises an auxiliary channel, and the auxiliary channel is watertight.

8. The electronic endoscope according to claim 7, wherein an intersection of the auxiliary channel and the end face of the distal end of the front end portion is located in an area outside the inclined surface.

9. The electronic endoscope according to claim 7, wherein two auxiliary channels are provided; and intersections of the two auxiliary channels and the end face of the distal end of the front end portion are respectively close to the two lighting units.

10. The electronic endoscope according to claim 1, wherein the insertion portion further comprises a flexible tube portion; each of the front end portion, the bending portion and the flexible tube portion is provided with an axially run-through working channel; and the working channels of the front end portion, the bending portion and the flexible tube portion communicate with one another through pipe fittings.
